# EUROPEAN PATENT APPLICATION

(11) **EP 4 016 797 A1**
(43) Date of publication of application: **22.06.2022**
(21) Application number: 20214373.1
(22) Date of filing: 15.12.2020
(51) Int. Cl.: H02J 50/12, A61N 1/378, H02J 50/80, H02J 50/23, H02J 50/27

(54) **WIRELESS POWER TRANSFER SYSTEMS FOR AN IMPLANTABLE MEDICAL DEVICE**

(71) Applicant: Capri Medical Limited, Dublin D04 W2K0 (IE)
(72) Inventor: Usman, Muhammad, Dublin, D04 W2K0 (IE); Assambo, Cédric, Dublin, D04 W2K0 (IE); O'Keeffe, Mike, Dublin, D04 W2K0 (IE); Mullins, John, Dublin, D04 W2K0 (IE); Ring, Tim, Dublin, D04 W2K0 (IE); Abbas, Farhat, Dublin, D04 W2K0 (IE); Ward, Fergal, Dublin, D04 W2K0 (IE)
(74) Representative: HGF

(57) **Abstract**

A wireless power transfer system for an implantable medical device is disclosed. The system comprises a transmitter configured to transmit wireless power transmission at a first operating frequency, and a receiver configured to receive the transmitted wireless power transmission, and to be electrically connectable to an implantable medical device. The transmitter is further configured to receive from the receiver an indication of an efficiency of the wireless power transmission at the first operating frequency, and if the efficiency falls below a threshold, then transmit wireless power transmission at a second operating frequency different to the first operating frequency, wherein the transmitter and receiver are configured to operate in any of regions; near-field, mid-field and far-field.

## Description

The present disclosure relates to wireless power transfer systems, transmitter antennas and receiver antennas suitable for use with an implantable medical device.

### BACKGROUND

Implantable medical devices may require a power source in order to operate and deliver the desired therapeutic effect and/or diagnostic effect. The power source is typically a rechargeable battery forming part of the implantable medical device that can be recharged wirelessly when in proximity of a charging station. For the wireless charging to be effective, the patient needs to stay within the proximity of the charging station until the rechargeable battery has been recharged.

### BRIEF SUMMARY OF THE DISCLOSURE

According to the present disclosure, there is provided a wireless power transfer system for an implantable medical device. The system comprising a transmitter configured to transmit wireless power transmission at a first operating frequency, a receiver configured to receive the transmitted wireless power transmission, and to be electrically connectable to an implantable medical device, the transmitter being further configured to; receive from the receiver an indication of an efficiency of the wireless power transmission at the first operating frequency, and if the efficiency falls below a threshold, then transmit wireless power transmission at a second operating frequency different to the first operating frequency, wherein the transmitter and receiver are configured to operate in any of regions; near-field, mid-field and far-field.

The wireless power transfer system enables the efficiency to be controlled. For example, when a user implanted with a medical device comprising the receiver moves relative to the transmitter, the efficiency can be maintained or increased, and in some examples, it can be enable the wireless power transfer to not fall below a particular efficiency, or be discontinued.

The transmitter and the receiver are configured to operate in the following frequency bands; the ultra-high frequency band (UHF), L-band, S-band, C-band and X-band. This means that the system is adaptable and can operate across 30MHz to 12GHz such that the wireless power transfer is flexible with regards to the distance between the receiver and the transmitter.

In one example the transmitter may comprise a transmitter antenna as claimed in any claims 12 to 17, and as described below. The transmitter is configured to transmit the wireless power transmission.

In one example, the receiver may comprise a receiver antenna as claimed in any of claims 19 to 22, wherein the receiver is configured to receive the wireless power transmission.

In one example, the transmitter may be configured to, prior to transmitting wireless power transmission at a second operating frequency, increase the transmit power for transmitting the wireless power transmission at the first operating frequency.

This gives the system flexibility in how to handle efficiency of the wireless power transfer.

In one example, the transmitter is configured to determine the second operating frequency based on the efficiency.

In one example, the receiver can be configured to determine power information comprising a value of the power received from the wireless power transmission, and send the power information to the transmitter, the transmitter being further configured to calculate the efficiency of the wireless power transfer based on the power information. This may occur periodically so that the system can adapt to a user moving whilst their implantable medical device is being wirelessly powered and/or charged.

In one example, the receiver is configured to periodically send the power information to the transmitter over a wireless communication interface so that the operating frequency can be continuously adjusted.

In one example, the receiver is configured to receive transmitted wireless power transmission from the transmitter over a distance from 1 cm to 100cm. The system enables the wireless power transfer to be performed across this distance and as such the system can operate in near-field, mid-field and far-field.

In one example, the receiver forms part of an implantable medical device. In one example, the implantable medical device is a neurostimulation device.

In one example, the transmitter may be further configured to compare the efficiency with a second threshold, such that the first threshold and the second threshold form a range, and wherein a range is associated with a transmit power and/or operating frequency.

In one example, the transmitter may be further configured to compare the efficiency with a second threshold, such that if the efficiency exceeds the second threshold, the transmitter reduces the transmit power and/or operating frequency. This can be advantageous if the system wants to save on power of the transmitter, and/or if to limit the transmitter output power to remain below Specific Absorption Rate (SAR) limit of 1.6W/Kg.

According to another aspect of the disclosure, there is provided a transmitter antenna for transmitting wireless power transmission to a receiver in near-field, mid-field and far-field. The transmitter antenna comprises a radiator patch and two ground patches located on a substrate such that the radiator patch and ground patches are coplanar, each ground patch being connected to the radiator patch via a pin-diode switch such that the radiator patch can be electrically connected to each ground patch independently.

This transmitter antenna may be referred to as a reconfigurable transmitter antenna.

The substrate may comprise a glass-reinforced epoxy resin laminate and the radiator patch and ground patches may comprise copper. The radiator patch and the ground patches may be block shaped and the ground patches partially surround the radiator patch. The substrate may be 35mm wide, 35mm long, and 1mm thick.

According to another aspect of the disclosure, there is provided a transmitter antenna for transmitting wireless power transmission to a receiver in near-field, mid-field and far-field. The transmitter antenna comprises a radiator patch located on one side of a substrate, and a ground patch located on an opposing side of the substrate, and the radiator patch having a meandering end opposing a narrowing end.

This antenna may be referred to as a multiband antenna.

The substrate may comprise a glass-reinforced epoxy resin laminate and the radiator patch and the ground patch may comprise copper.

Any of the transmitters may be configured to transmit wireless power transmission in the following frequency bands; the ultra-high frequency band (UHF), L-band, S-band, C-band and X-band.

According to another aspect of the disclosure, there is provided a charging unit comprising the transmitter antenna as claimed in any of claims 12 to 17. The charging unit may be referred to as an External Power Unit (EPU) and it may be a rechargeable wearable, or it may be stationary unit.

According to another aspect of the disclosure, there is provided a receiver antenna for receiving wireless power transmission from a transmitter in near-field, mid-field and far-field. The receiver antenna comprising an inner helix located on a disc, and an outer helix surrounding the inner helix, wherein the outer helix comprises electrical terminals.

In one example, the outer helix extends in an axial direction beyond that of the inner helix. The outer helix may be positioned on or around the disc.

In one example, the outer helix is 5.5mm in height and has a diameter of 3mm, and the inner helix is 3mm in height and has a diameter of 1.5mm.

According to one aspect of the disclosure, there is provided an implantable medical device comprising a receiver antenna as claimed in any of claims 19 to 22.

According to one aspect of the disclosure, there is provided a receiver comprising a receiver antenna as claimed in any of claims 19 to 22.

According to one aspect of the disclosure, there is provided a transmitter comprising a transmitter antenna as claimed in any of claims 12 to 17.

Within the scope of this application it is expressly intended that the various aspects, embodiments, examples and alternatives set out in the preceding paragraphs, in the claims and/or in the following description and drawings, and in particular the individual features thereof, may be taken independently or in any combination. That is, all embodiments and/or features of any embodiment can be combined in any way and/or combination, unless such features are incompatible. The applicant reserves the right to change any originally filed claim or file any new claim accordingly, including the right to amend any originally filed claim to depend from and/or incorporate any feature of any other claim although not originally claimed in that manner.

### BRIEF DESCRIPTION OF THE DRAWINGS

Various features of the present disclosure will be apparent from the detailed description which follows, taken in conjunction with the accompanying drawings, which together illustrate features of the present disclosure, and wherein:
Figure 1 is a schematic illustration of a wireless power transfer system according to the disclosure;
Figure 2 is a schematic illustration of an example transmitter according to the disclosure;
Figure 3 is a schematic illustration of an example receiver according to the disclosure;
Figure 4 is a schematic illustration of another example transmitter according to the disclosure;
Figure 5 is a schematic illustration of an example transmitter antenna operable with the transmitter of figure 2 according to the disclosure;
Figure 6 is a schematic illustration of an example transmitter antenna operable with the transmitter of figure 4 according to the disclosure; and
Figure 7 to 9 is a schematic illustration of an example receiver antenna according to the disclosure operable with the receiver of figure 3;
Figure 10 is a schematic illustration of an example wireless power transfer system;
Figure 11 is a schematic illustration of an example charging unit comprising an example transmitter; and
Figure 12 illustrates an example method of a wireless power transfer system.

### DETAILED DESCRIPTION

Implantable medical devices requiring energy to operate typically comprise a rechargeable battery as their power source. This eliminates the need for the user to be continuously connected to the mains. The battery of implantable medical devices may be recharged via wireless power transfer (WPT) as this avoids the need for physical links between the implantable medical device and the power source. WPT comprises a transmitter device transmitting electrical energy to a receiver in the implantable medical device. However, the efficiency of WPT is dependent on the proximity of the implantable medical device, thus the user, to the transmitter device. The efficiency of WPT is also affected by the orientation of the implantable medical device relative to the transmission device, in that a slight movement of the user can cause loss of WPT. Thus, a user wishing to use or recharge their implanted medical device will typically have to stay near the transmitter device without significantly moving for an extended period of time. This may not always be convenient to a user, in particular, if they suffer from pain by staying in the same position.

An example of an implantable medical device is an implantable neurostimulation system that target specific deep subcortical, cortical, spinal, cranial, and peripheral nerve structures, for treatment of pain in patients. Neurostimulation is an alternative to pain medication and nerve block injections. It is associated with fewer side effects than many medications and can reduce the potential for drug dependency.

The present disclosure provides examples of how an implantable medical device, like a neurostimulation system, can be configured such that the wireless power transfer can be effective regardless of the near distance and/or orientation of a user of an implanted medical device relative to the transmitter device.

It should be understood that the implantable medical device described herein may be any medical device that is implantable in a human being or animal, and that requires electricity to operate. An example of an implantable medical device is a neurostimulation device.

In the following description, for purposes of explanation, numerous specific details of certain examples are set forth. Reference in the specification to "an example" or similar language means that a particular feature, structure, or characteristic described in connection with the example is included in at least that one example, but not necessarily in other examples.

The following description relates to electrically small antennas, meaning that the antennas herein have a physical size of less than the wave length of the operating frequency. In view of this, the terms near-field, mid-field and far-field can be considered as different operating regions depending on the operating frequency and a distance between a transmitter antenna and a receiver antenna. Near-field may be considered when the distance between the transmitter antenna and the receiver antenna is less than one wave length of the operating frequency, mid-field may be considered when the distance between the transmitter antenna and the receiver antenna is close or on the boarder of one wavelength of the operating frequency, and far-field may be considered when the distance between the transmitter antenna and receiver antenna is more than one wave length of the operating frequency.

As disclosed herein, "operating frequency" is to be understood as the frequency in which a wireless power transfer system, transmitter, receiver, transmitter antenna and/or receiver antenna are operating at. For example, the operating frequency is the frequency of radio waves transmitted by a transmitter and received by a receiver.

Examples disclosed herein relate to wireless power transfer systems, transmitters, receivers, transmitter antennas and receiver antennas that are configured to enable wireless power transmission with the view to recharge a battery forming part of an implantable medical device. The examples enable a user implanted with a medical device to move relative to a charging unit without significant detriment to the efficiency of the wireless power transfer. The examples may also, or alternatively, enable the transmit power and/or operating frequency of the transmitter antenna to be controlled.

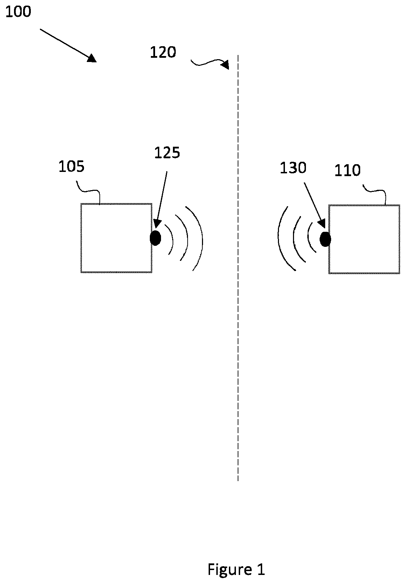

Figure 1 shows a schematic representation of a wireless power transfer system 100 for an implantable medical device 105. The implantable medical device 105 is located underneath the skin 120 of a user at some depth depending on the type of implantable medical device. The system 100 further comprises a charging device 110 located outside of the body of the user. The wireless power transfer system 100 comprises a transmitter 130 which is configured to transmit wireless power transmission to the implantable medical device, such that a receiver 125 of the medical implantable medical device detects the wireless power transmission and harvests the energy of the power transmission. The energy received by the receiver is then stored in a rechargeable battery.

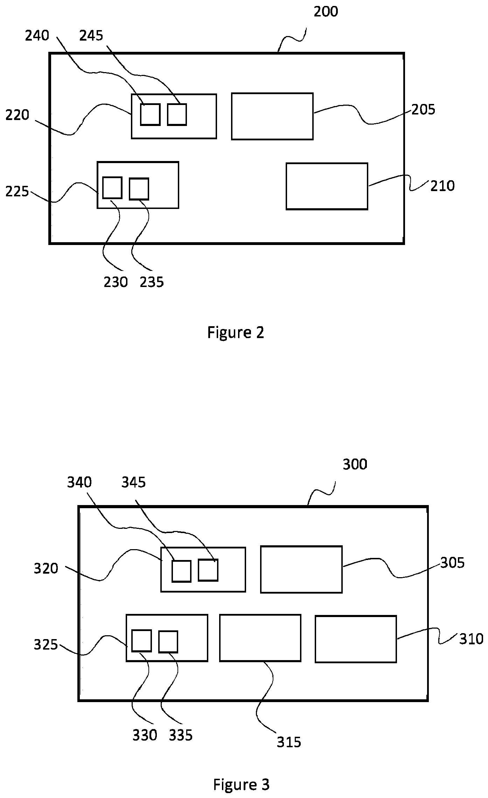

An example transmitter that can form part of a charging unit and an example receiver that can form part of an implantable medical device will now be described with reference to figures 2 to 4.

Figure 2 shows a schematic representation of an example transmitter 200. The transmitter 200 is for transmitting energy in the form of radio waves and thereby powering and/or charging an implantable medical device wirelessly. The transmitter 200 is configured to operate in different frequencies depending on the distance and/or orientation between the transmitter 200 and a receiver forming part of the implantable medical device. The operating range of the transmitter is 30MHz to 12GHz and the receiver may be any receiver that is capable of receiving and harvesting energy from radio waves in the region of 30MHz to 12GHz. An example of such a receiver is described below with reference to figure 3. By changing the frequency of the radio waves transmitted by the transmitter 200, the wireless power transmission can be maintained, improved or optimized in dependence with the distance and/or orientation between the transmitter 200 and the receiver. This can also be described as by changing the frequency of the radio waves as the distance and/or orientation change between the transmitter 200 and the receiver, the loss of the wireless power transmission can be controlled. The transmitter 200 and the receiver are configured in such a way that they can operate in any of regions; near-field, mid-field and far-field.

The transmitter 200 comprises an electronic circuit 225 comprising a processor 230 and a memory 235. The electronic circuit may also be referred to as an external processing unit (EPU) electronic circuit and it electrically connects all the components of the transmitter 200. The memory 235 comprises instructions executable by the processor 230 such that the transmitter 200 is operable to control the components of the transmitter 200 and perform the methods described below. The components controlled by the processor 230 include at least an adaptive gain control circuit, a transmitter antenna, a voltage-controlled oscillator and a communication unit each of which is described below.

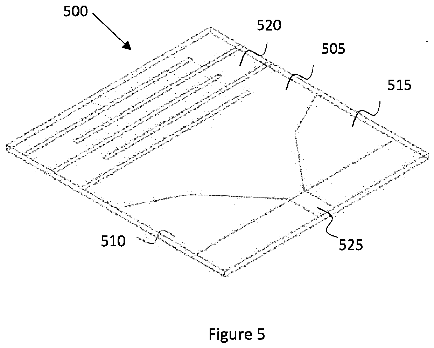

The transmitter 200 comprises a transmitter antenna 205 for transmitting energy in the form of electromagnetic waves in the radio frequency range. The transmitter antenna is a multiband transmitter antenna meaning that it can transmit radio waves in different frequency bands including ultra-high frequency (UHF), L-band, S-band, C-band and X-band. The frequency range of each band may be defined as follows; UHF is 30MHz to 1GHz, L-band is 1-2GHz, S-band is 2-4GHz, C-band is 4-8 GHz and X-band is 8-12GHz. The frequency transmitted by the transmitter antenna 205 can be changed by a voltage-controlled oscillator (VCO) which, based on an instruction from the processor 230, can change the oscillation of the current such that the frequency of the transmitter antenna 205 changes to a desired frequency. An example transmitter antenna used with the transmitter 200 is described below with reference to figure 5.

The transmitter 200 further comprises an adaptive gain control circuit 210 for adjusting the transmit power and/or changing the operating frequency of the radio waves transmitted by the transmitter antenna 205. The adaptive gain control circuit 210 is configured to receive power information comprising an indication of the power received by the receiver from the transmitter 200 during wireless power transfer. Based on this information, the adaptive gain control circuit 210 calculates the efficiency of the wireless power transfer. Using the efficiency, the adaptive gain control circuit 210 determines whether to increase or decrease the transmit power used for transmitting the radio waves via the transmitter antenna 205. The adaptive gain control circuit 210 is also configured to determine, based on the calculated efficiency, whether to change the operating frequency for the wireless power transfer of the transmitter 200. The calculated efficiency of the received power in relation to the operating frequency may alternatively be referred to as an indication of efficiency or an efficiency value.

The transmitter 200 further comprises a communication unit 220. The communication unit 220 is for communicating with the receiver. The communication unit comprises a communication interface 240 and a communication circuit 245. The communication interface 240 is configured to receive/transmit signals comprising information, over an air interface, to/from the receiver. The communication interface 240 may be configured to send and receive data via Bluetooth Low Energy (BLE). BLE is a protocol used for medical device communications. The BLE communication protocol utilises low power consumption and easily synchronizes with connected devices. Other suitable wireless communication technology that can be implemented include Medical Implant Communication System (MICS), Zigbee or WiFi. The communication circuit 245 may be an external processing unit (EPU) communication circuit. The communication circuit 245 may be a BLE circuit. The communication circuit 245 is configured to transmit/receive signals to/from the electronic circuit 225 as well as the communication interface 240. When receiving a signal from the receiver over an air interface, the communication interface 240 sends the received information to the communication circuit 245 which forwards the received information to the processor 230 of the electronic circuit. When information is to be sent from the transmitter 200 to the receiver, the electronic circuit 225 sends a signal to the communication circuit 245 which in turn sends the signal to the communication interface 240 which transmits the information over an air interface to the receiver. The signals may be considered to comprise information, for example, a message to be sent via the communication interface 240, including the address and content of such a message, and appropriate authentication information if needed. Examples of such a message include a query message from the transmitter 200 to the receiver asking for information on the power received by the receiver or the battery status of the implantable medical device. In another example, the communication unit 220 is also operable to receive power information from the receiver such as information relating to the power received by the receiver from the transmitter 200 during wireless power transfer. The communication unit 220 will then proceed to relay the information to the electronic circuit 225 which then calculates efficiency of the wireless power transfer as described above.

The transmitter 200 further comprises a power source which may be mains or battery. The power source is connected to the electronic circuit 225 which powers all of the components of the transmitter 200. In one example, the transmitter 200 may be connected to a wearable (rechargeable) battery giving the user even more flexibility whilst the implantable medical device is being used and/or charged.

In the transmitter 200 a single common processer is described, namely processor 230, for controlling the transmitter and all of its components. This may be referred to as a centralised architecture. However, it should be understood that a more distributed architecture may be adopted where each of the adaptive gain control circuit 210 and communication circuit 220 have their own processor. It is also possible for the transmitter 200 to have a combination of a centralised and distributed architecture in terms of processors and circuits.

In one example, the transmitter 200 forms part of a charging unit. The charging unit may be a stationary unit connected to the mains or battery driven. Alternatively, the charging unit may be a wearable device comprising a (rechargeable) battery such as a wristband or a device that can be carried in a pocket.

Figure 3 will now be described which shows a schematic illustration of a receiver 300. The receiver 300 forms part of an implantable medical device, or it may be considered to be electrically connected to an implantable medical device. The receiver 300 is for receiving power, or more specifically, radio waves transmitted by the transmitter, and harvesting the energy of the radio waves. The received energy can be stored in a battery or it can be used instantly by the implantable medical device. As mentioned above, the receiver 300 and the transmitter 200 are configured in such a way that they can operate in any of regions; near-field, mid-field and far-field.

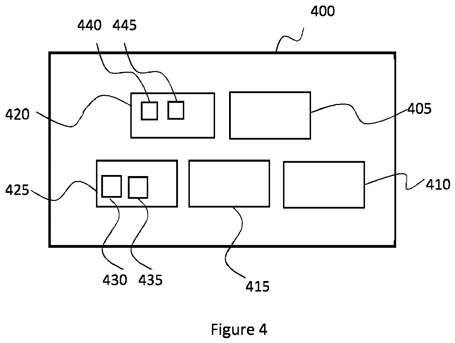

It should be understood that the receiver 300 is not limited to receive power from the transmitter 200 described with reference to figure 2. In an alternative example, the receiver 300 is configured to receive power from a transmitter 400 as described with reference to figure 4. In fact, the receiver 300 can receive power from any transmitter operating in the frequency range of 30MHz to 12 GHz. However, for the sake of legibility of this description, only the transmitter 200 will be referred to below with reference to figure 3.

The receiver 300 can receive radio waves having a frequency ranging at least between 30MHz to 12GHz. Thus, by the transmitter 200 changing the operating frequency, the wireless power transfer can be optimized in dependence with the distance and/or orientation between the transmitter 200 and the receiver 300.

The receiver 300 comprises an electronic circuit 325 comprising a processor 330 and a memory 335. The memory 335 stores instructions executable by the processor 330 such that the processor performs the methods described below. In other words, the electronic circuit 325 electrically connects all the components of the receiver including at least a receiver antenna, converter, communication unit and a voltage-controlled oscillator each of which is described below. The processor 330 can be described to control the components of the receiver 300.

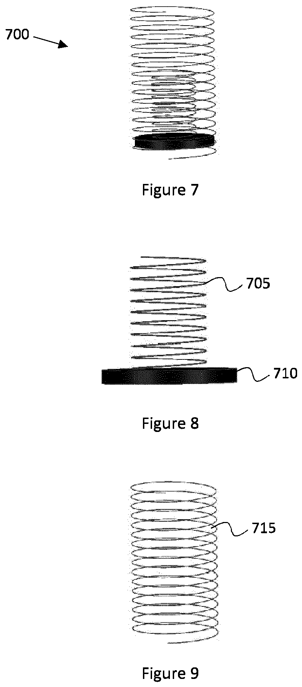

The receiver 300 further comprises a receiver antenna 305. The receiver antenna 305 is for receiving energy in the form of radio waves in the radio frequency range transmitted by the transmitter 200. The receiver antenna 305 is a multiband receiver antenna, and it is a passive antenna acting as a passive transducer for harvesting the energy of the radio waves transmitted by the transmitter 200. The resonance of the receiver antenna 305 is defined by its geometry and material properties. An example receiver antenna is described below with reference to figures 7 to 9.

The receiver antenna 305 further comprises a converter 310 for converting received radio frequency to direct current. This may also be referred to as a RF to DC circuit. As the receiver antenna 305 receives the radio waves in the operating frequency of the transmitter 200, alternating current is produced in the receiver antenna 305. The converter 310 converts the alternating current to direct current. The value or measurement of the direct current received is used as power information sent to the transmitter 200 to indicate the power received by the receiver 300. The transmitter 200 uses the power information to calculate or determine efficiency of the wireless power transfer as described above.

The power received by the receiver antenna 305 may be used instantly to operate an implantable medical device that it is electrically connected with, and/or it may be stored in a rechargeable battery 315 connected to the electronic circuit 325 of the receiver 300 such that the implantable medical device can use the power later on.

The receiver 300 also comprises a communication unit 320 having a communication interface 340 and a communication circuit 345. The communication unit 320 is for communicating with the transmitter 200. The communication unit 320 may be configured to send and receive data via Bluetooth or other wireless communication technology. The communication circuit 345 may be a BLE (Bluetooth low energy) circuit, and the communication interface 340 may be a BLE interface. Other suitable wireless communication technology that can be implemented include Medical Implant Communication System (MICS), Zigbee or WiFi. The communication interface 340 is configured to receive/transmit signals comprising information, over an air interface, to/from the transmitter 200. When receiving a signal from the transmitter 200, the communication interface 340 sends the received information to the communication circuit 345 which forwards the received information to the processor 330 of the electronic circuit. The communication circuit 345 is also configured to receive information from the electronic circuit 325, for example power information which is information on the power the receiver 200 received from the transmitter 200 during wireless power transfer. The communication circuit 345 is configured to transmit the power information via the communication interface 340 to the communication interface 240 of the transmitter 200.

In the receiver 300 a single common processor is described, namely processor 330, for controlling the receiver and all of its components. This may be considered to be a centralised architecture. However, it should be understood that a more distributed architecture may be adopted where each of the communication circuit 420 and converter 310 have their own processor. It is also possible for the receiver 300 to have a combination of a centralised and distributed architecture in terms of processors and circuits.

Although the receiver 300 has been described as receiving wireless power transmission from transmitter 200, it should be understood that it can receive power transmission from any transmitter transmitting in the range of 30MHz - 12 GHz. Another example transmitter will now be described with reference to figure 4 and also this transmitter is operable with the receiver 300.

Figure 4 shows a schematic illustration of another example transmitter 400. This example transmitter 400 is an alternative transmitter to the transmitter 200 described with referent to figure 2.

The transmitter 400 is for transmitting energy in the form of radio waves and thereby powering or charging an implantable medical device wirelessly. The transmitter 400 is configured to operate in different frequencies depending on the distance and/or orientation between the transmitter 400 and a receiver forming part of the implantable medical device. The operating range of the transmitter is 30MHz to 12GHz. By changing the frequency of the radio waves transmitted by the transmitter 400, the wireless power transmission can be maintained, improved or optimized in dependence with the distance and/or orientation between the transmitter 400 and the receiver. This can also be described as by changing the frequency of the radio waves as the distance and/or orientation change between the transmitter 400 and the receiver, the loss of the wireless power transmission can be controlled. The transmitter 400 and the receiver are configured in such a way that they can operate in any of regions; near-field, mid-field and far-field. The receiver may be any receiver that is capable of receiving and harvesting energy from radio waves in the region of 30MHz to 12GHz. The receiver 300 described with reference to figure 3 may be such a receiver and for the ease of legibility, receiver 300 will be used in the description below of the transmitter 400.

The transmitter 400 comprises an electronic circuit 425 comprising a processor 430 and a memory 435. The electronic circuit may also be referred to as an external processing unit (EPU) electronic circuit and it electrically connects all the components of the transmitter 400. The memory 435 comprises instructions executable by the processor 430 such that the transmitter 400 is operable to control the components of the transmitter 400 and perform the methods described below. The components controlled by the processor 430 include at least a transmitter antenna, an adaptive gain control circuit, switching circuit 415, a voltage-controlled oscillator (VCO) and a communication unit, each of which is described below.

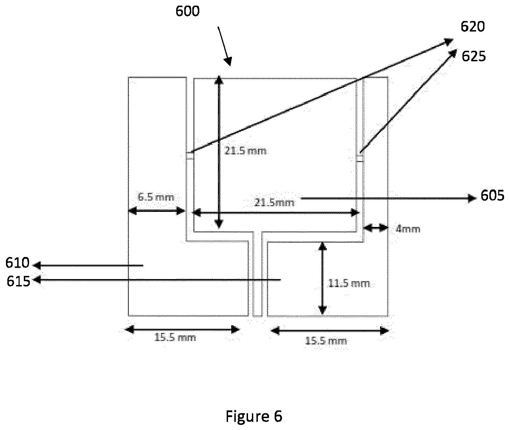

The transmitter 400 comprises a transmitter antenna 405 for transmitting energy in the form of electromagnetic waves in the radio frequency range. The transmitter antenna 305 is a reconfigurable transmitter antenna meaning that it can transmit radio waves in different frequency bands including ultra-high frequency (UHF), L-band, S-band, C-band and X-band. The frequency range of each band may be defined as follows; UHF is 30MHz to 1GHz, L-band is 1-2GHz, S-band is 2-4GHz, C-band is 4-8 GHz and X-band is 8-12GHz. An example transmitter antenna used with the transmitter 400 is described below with reference to figure 6 which shows that the resonance frequency of the transmitter antenna can be changed by activating/deactivating pin-diodes. The operating frequency of the radio waves transmitted by the transmitter antenna 405 is then changed by a voltage-controlled oscillator, forming part of the electronic circuit, which, based on an instruction from the processor 425, can change the oscillation of the current such that the operating frequency of the transmitter antenna 405 changes to a desired frequency.

The transmitter 400 further comprises an adaptive gain control circuit 410 for adjusting the transmit power and/or changing the operating frequency of the transmitter antenna 405. The adaptive gain control circuit 410 is configured to receive power information comprising an indication of the power received by the receiver from the transmitter during wireless power transfer. Based on this information, the adaptive gain control circuit 410 calculates the efficiency of the wireless power transfer. Using the efficiency, the adaptive gain control circuit 410 determines whether to increase or decrease the transmit power used for transmitting the radio waves via the transmitter antenna 405. The adaptive gain control circuit 410 is also configured to determine, based on the calculated efficiency, whether to change the operating frequency for the wireless power transfer of the transmitter. The calculated efficiency of the received power in relation to the operating frequency may alternatively be referred to as an indication of efficiency or an efficiency value.

The transmitter 400 also comprises a switching circuit 415 as mentioned above. The switching circuit enables and disables different parts of the transmitter antenna geometry by activating or deactivating pin diodes connecting a radiator patch with two ground patches of the transmitter antenna. This is to change the resonance frequency of the transmitter antenna for wireless power transfer as determined by the adaptive gain control circuit 410.

The transmitter 400 further comprises a communication unit 420. The communication unit 420 is for communicating with the receiver 300. The communication unit 420 comprises a communication interface 440 and a communication circuit 445. The communication interface 440 is configured to receive/transmit signals comprising information, over an air interface, to/from the receiver. The communication interface 440 may be configured to send and receive data via Bluetooth Low Energy (BLE). Other suitable wireless communication technology that can be implemented include Medical Implant Communication System (MICS), Zigbee or WiFi. The communication circuit 445 may be an external processing unit (EPU) communication circuit. The communication circuit 445 may be a BLE circuit. The communication circuit 445 is configured to transmit/receive signals to/from the electronic circuit 425 as well as the communication interface 440. When receiving a signal from the receiver over an air interface, the communication interface 440 sends the received information to the communication circuit 445 which forwards the received information to the processor 430 of the electronic circuit. When information is to be sent from the transmitter 400 to the receiver, the electronic circuit 425 sends a signal to the communication circuit 445 which in turn sends the signal to the communication interface 440 which transmits the information over an air interface to the receiver. The signals may be considered to comprise information, for example, a message to be sent via the communication interface 440, including the address and content of such a message, and appropriate authentication information if needed. Examples of such a message include a query message from the transmitter 400 to the receiver asking for information on the power received by the receiver or battery status of the implantable medical device. In another example, the communication unit 420 is also operable to receive information from the receiver such as power information relating to the power received by the receiver from the transmitter 400. The communication unit 420 will then proceed to relay the information to the electronic circuit 425.

The transmitter 400 further comprises a power source which may be mains or battery. The power source is connected to the electronic circuit 425 which powers all of the components in the transmitter. In one example, the transmitter 400 may be connected to a wearable (rechargeable) battery giving the user even more flexibility whilst the implantable medical device is being charged.

In the transmitter 400 a single common processor is described, namely processor 430, for controlling the transmitter and all of its components. This may be considered to be a centralised architecture. However, it should be understood that a more distributed architecture may be adopted where each of the adaptive gain control circuit 410, switching circuit 415 and communication circuit 420 have their own processor. It is also possible for the transmitter 400 to have a combination of a centralised and distributed architecture in terms of processors and circuits.

In one example, the transmitter 400 forms part of a charging unit. The charging unit may be a stationary unit connected to the mains or battery driven. Alternatively, the charging unit may be a wearable device that comprises a (rechargeable) battery such as a wristband or a device that can be carried in a pocket.

Figure 5 shows a schematic illustration of a transmitter antenna 500. The transmitter antenna 500 may be referred to as a multiband transmitter antenna. The transmitter antenna 500 is for transmitting wireless power transmission to a receiver in near-field, mid-field and far-field. The transmitter antenna 500 is connectable to a power source such as mains or a battery providing alternating current to the transmitter antenna 500. When the transmitter antenna 500 is electrically connected, it can transmit radio waves in any of the following frequency bands; UHF (30MHz to 1GHz), L-band (1-2GHz), S-band (2-4GHz), C-band (4-8 GHz) and X-band (8-12GHz). In near-field and mid-field, power is transferred from the transmitter antenna 500 to the receiver antenna via magnetic fields by inductive coupling. In the far-field, power is transmitted or radiated by the transmitter in the form of electromagnetic waves. An example receiver antenna operable with the transmitter antenna 500, may be the receiver antenna 700 described below with reference to figures 7 to 9.

As seen in figure 5, the transmitter antenna 500 is of a flat or patch design. It comprises a radiator patch 505 located on one side 515 of a substrate 510. The radiator patch 505 has a meandering end 520 opposing a narrowing end 525. The narrowing end 525 has a funnel or a Y-shape. On an opposing side of the substrate 510, a ground patch is located (not shown). Thus, the transmitter antenna 500 has a layered structure wherein the substrate 510 is sandwiched between the ground patch and the radiator patch 505. The ground patch is at zero potential with respect to the radiator patch 505. The radiator patch 505 can be electrically connected to an electronic circuit so as to receive alternating current resulting in radio waves being transmitted by the transmitter antenna 500.

The radiator patch 505 and the ground patch may both be made of copper. The substrate 510 may be made of a glass-reinforced epoxy laminate material that has a National Electrical Manufacturers Association (NEMA) grade designation of FR4. FR4 may be described as a composite material comprising woven fiberglass cloth with an epoxy resin binder that is flame resistant.

The transmitter antenna 500, and so also the substrate 510, may be of 45mm in length, 42mm in width. The substrate may be 1mm in thickness. The ground patch may be 45mm in length and 7mm in width.

Figure 6 shows a schematic illustration of another transmitter antenna 600. The transmitter antenna 600 may be referred to as a reconfigurable transmitter antenna. The transmitter antenna 600 is for transmitting wireless power transmission to a receiver in near-field, mid-field and far-field. The transmitter antenna 600 is connectable to a power source such as mains or a battery providing alternating current to the transmitter antenna 600. When the transmitter antenna 600 is electrically connected, it can transmit radio waves in any of the following frequency bands; UHF (30MHz to 1GHz), L-band (1-2GHz), S-band (2-4GHz), C-band (4-8 GHz) and X-band (8-12GHz). In near-field and mid-field, power is transferred from the transmitter antenna 600 to the receiver antenna via magnetic fields by inductive coupling. In the far-field, power is transmitted or radiated by the transmitter in the form of electromagnetic waves. An example receiver antenna operable with the transmitter antenna 500, may be the receiver antenna 700 described below with reference to figures 7 to 9.

The transmitter antenna 600 is of a patch or flat design. It has a defected structure that may be described as a modular structure. This means that it comprises a plurality of flat metal components. The metal components are electrically connected by pin-diodes such that that the various components can be activated and deactivated thereby changing the operating frequency of the whole antenna as will now be described.

More specifically, as seen in figure 6, the transmitter antenna 600 comprises a radiator patch 605 and two ground patches referred to as a first ground patch 610 and a second ground patch 615. The radiator patch 605 and the two ground patches 610, 615 are positioned on the same side of a substrate, such that they are coplanar. The radiator patch 605 and the ground patches 610, 615 are block-shaped, where the ground patches 610, 615 are formed as two mirroring L-shapes with the radiator patch 605 positioned in between. The dimensions of the radiator patch 605 and ground patches 610, 615 are shown in figure 6. The radiator patch 605 can be electrically connected to the ground patches 610, 615 by a first and second pin-diode 620, 625. The pin-diodes act like radio frequency switches and can be switched "on" and "off". Thus, four configurations are possible of the reconfigurable antenna. In a first configuration the two pin-diodes 620, 625 are "off" such that the radiator patch 605 is electrically disconnected from both ground patches 610, 615. In a second configuration the first pin-diode 620 is "on" and the second pin-diode 625 is "off' such that the radiator patch 605 is electrically connected to the first ground patch 610. In a third configuration the second pin-diode 625 is "on" and the first pin-diode 620 is "off' such that the radiator patch 605 is electrically connected to the second ground patch 615. In a fourth configuration both the pin-diodes 620, 625 are "on" such that the radiator patch 605 is electrically connected to both the first and the second ground patch 610, 615. In this manner, the resonance frequency of the transmitter antenna 600 can be changed according to the states of the pin-diodes. Additionally, when connected to a voltage-controlled oscillator, the operating frequency of the transmitter antenna can be changed to transmit a frequency of radio waves between 30MHz to 12GHz.

To explain the use of pin-diodes further, the two pin-diodes can be turned "on" and "off" due to direct current (DC) biasing voltage present between the radiator patch 605 and the ground patches 610, 615. (Whereas alternating current (AC) voltage carrying the RF signal is super imposed on the DC.) Under zero- or reverse-bias (the "off' state) a pin diode as a low capacitance. The low capacitance will not pass much of a radio frequency (RF) signal. Under a forward bias of 1mA (the "on" state), a pin-diode will have a radio frequency (RF) resistance of about 1ohm, making it a good conductor of RF. Thus, the pin-diode acts like a RF switch.

The dimensions of the substrate and so also the transmitter antenna 600 may be 35mm in length and 35mm in width. The substrate may be 1mm thick and may be made of a glass-reinforced epoxy laminate material that has a National Electrical Manufacturers Association (NEMA) grade designation of FR4. The radiator patch 605 and the ground patches 610, 615 may be made of copper.

Figures 7 to 9 shows a schematic illustration of receiver antenna 700. The receiver antenna 700 may be referred to as a multiband receiver antenna. The receiver antenna 700 is for receiving wireless power transmission from a transmitter antenna, such as transmitter antenna 500 or 600, in near-field, mid-field and far-field. The receiver antenna 700 is a passive antenna and so does not need power in order to operate. The resonant frequency of the receiver antenna 700 are achieved due to the physical design of the antenna geometry. Thus, the receiver antenna 700 can receive radio waves in any of the following frequency bands; UHF (30MHz to 1GHz), L-band (1-2GHz), S-band (2-4GHz), C-band (4-8 GHz) and X-band (8-12GHz). In near-field and mid-field, power is received by the receiver antenna 700 from the transmitter antenna via magnetic fields by inductive coupling. In the far-field, the receiver antenna 700 receives power by electromagnetic waves transmitted or radiated by the transmitter. The receiver antenna 700 is operable with transmitter antennas 500, 600 described above.

The receiver antenna 700 comprises an inner helix 705 located on or connected to a disc 710 as is best illustrated in figure 8. In one example, the inner helix 705 is attached or connected to the disc 710. The receiver antenna 700 further comprises an outer helix 715 surrounding the inner helix 705. The outer helix 715 may extend in an axial direction beyond that of the inner helix 705. This means that the length of the outer helix 715, in a longitudinal direction, may be longer than that of the inner helix 705. The outer helix 715 may be positioned on or around the disc 710. In one example, the outer helix 715 is attached or connected to the disc 710.

The outer helix 715 may comprise electrical terminals connectable to an electrical circuit of a receiver, for example receiver 300, such that the energy harvested from the received radio waves can be stored in a rechargeable battery and/or instantly be used for operating an implantable medical device.

The outer helix 715 may be 5.5mm in height and may have an inner diameter of 3mm. The inner helix 705 may be 3mm in height and may have an inner diameter of 1.5mm. The wire thickness of the outer and inner helix may be 0.2mm. The disc may have a thickness of 0.25mm and a diameter of 3mm. The outer helix 715, inner helix 705 and the disc 710 may all be made of copper.

The outer helix 715, inner helix 705 and the disc 710 may all be soldered together.

An example of how the transmitter 200 and the receiver 300 may operate will now be described with reference to figures 2 to 4. For the purposes of this description, the transmitter 200 comprises a multiband transmitter antenna, for example, the transmitter antenna 500 shown in figure 5, and the receiver 300 comprises a multiband receiver antenna, for example, the receiver antenna 700 shown in figures 7 to 9.

When the receiver 300 and the transmitter 200 are in a charging state, the transmitter 200 transmits wireless power transmission in the form of radio waves at an operating frequency, and the receiver 300 and transmitter 200 may operate in any of near-field, mid-field or far-field. The distance and/or orientation of the receiver 300 relative to the transmitter 200 may continuously change due to movement of the user implanted with an implantable medical device comprising the receiver 300, thus in order to improve or maintain wireless power transfer, or reduce loss of wireless power transfer, the transmit power and/or operating frequency of the transmit antenna 205 will be changed as described below;

When the receiver 300 receives radio waves from the transmitter 200 and converts it into direct current, the receiver 300 measures a value of the direct current, power information, and sends this to the transmitter 200 via its communication unit 320. The transmitter 200 receives the power information via its communication unit 220 and sends it to the adaptive gain control circuit 210. The adaptive gain control circuit 210 determines a value of how effective the wireless power transfer was from the transmitter 200 to the receiver 300 based on the received power information. This value may be referred to as efficiency. The receiver 300 may send power information to the transmitter 200 periodically, for example every 0.1,1 or 60 second, and the adaptive gain control circuit 210 may therefore continuously determine the efficiency of the wireless power transfer based on the received power information and assess whether the transmit power and/or operating frequency of the transmitter 200 need to change. The adaptive gain control circuit 210 compares the efficiency with a threshold value and if the efficiency falls below the threshold value then the adaptive gain control circuit 210 will proceed to change transmit power and/or operating frequency. (The efficiency falling below the threshold value could be due to an increased distance and/or change in orientation between the transmitter 200 and the receiver 300). In one example, the adaptive gain control circuit 210 may first attempt to increase transmit power of the transmitter antenna 205 and if based on an updated power information received from the receiver 300 after the transmit power has been increased the efficiency value still falls below a threshold, the adaptive control circuit 210 may proceed with changing the operating frequency of the transmitter antenna 205. This process may be continuously repeated in order to find an optimal transmit power and/or frequency and also to adapt to the changing distance between the transmitter and receiver due to movement of the user relative to the transmitter.

In an alternative example on how the adaptive gain control circuit 210 handles the efficiency, the adaptive gain control circuit 210 compares the efficiency with a plurality of predetermined ranges of efficiency, each range relating to a transmit power and operating frequency. Thus, when the efficiency is determined to fall within a predetermined range associated with a transmit power and operating frequency, the adaptive gain control circuit 210 changes the transmit power and operating frequency of the transmitter antenna accordingly. This process may be continuously repeated to adapt to the changing distance between the transmitter 200 and receiver 300 due to movement of the user relative to the transmitter.

In yet another example, it may be that the adaptive control circuit 205 proceeds to change the operating frequency when the efficiency falls above a threshold value. For example, the operating frequency can be changed to lower the energy used in order to save battery or to limit the transmitter output power to remain below SAR limit of 1.6W/Kg.

The threshold and predetermined ranges of efficiency and associated transmit power and operating frequency of the transmit power may all be stored in the memory 235 of the electronic circuit 225.

When the adaptive gain control circuit 210 determines that the operating frequency needs to change, the adaptive gain control circuit 210 signals to the processor 230 which in turn instructs the voltage-controlled oscillator connected to the transmitter antenna 205 to increase or decrease the oscillation of the current in order to achieve the desired frequency transmitted by the transmitter antenna.

In another example, where the transmitter 400 and the receiver 300 are used for wireless power transfer, the receiver 300 and the transmitter 400 may operate on the same principle as described above wherein the receiver continuously determines power information and sends it over to the transmitter 400 which continuously determines the efficiency of the wireless power transfer, and then changes the transmit power and/or operating frequency in order to improve, maintain or control the wireless power transfer. However, when the transmitter 400 is used, its switching circuit 415 activates and/or deactivates relevant pin-diodes of the transmitter antenna described with reference to figure 6 in order to change the resonance frequency of the transmitter antenna, and then changing the operating frequency using the voltage-controlled oscillator.

In the above described examples, the receiver is configured to determine power information which indicates a value of the power received from the transmission of radio waves from the transmitter, and the transmitter is configured to determine an efficiency (or efficiency value) based on the power information. However, it should be understood that the receiver can instead be configured to also determine the efficiency based on the power information and then send the efficiency over to the transmitter such that the transmitter then compares the received efficiency with a threshold and/or predetermined ranges.

Examples of wireless power transfer systems will now be described. These examples may implement any combination of the features described above with reference to figures 2 to 9.

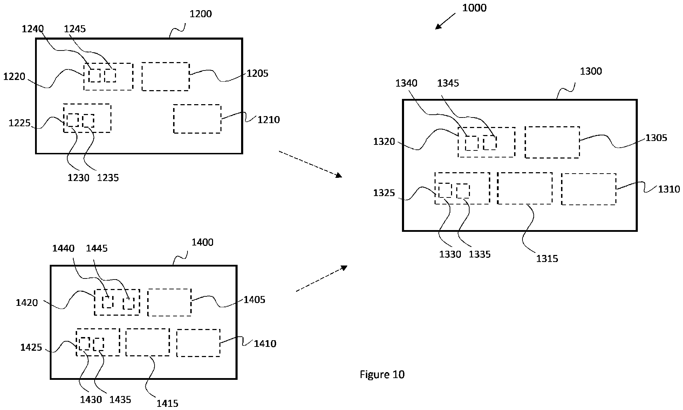

An example wireless transfer system 1000 will now be described with reference to figure 10. The wireless power transfer system 1000 is suitable for an implantable medical device. The system comprises a transmitter 1200, 1400 configured to transmit wireless power transmission at a first operating frequency, a receiver 1300 configured to receive the transmitted wireless power transmission, and to be electrically connectable to an implantable medical device. The transmitter 1200, 1400 is further configured to receiver from the receiver 1300 an indication of an efficiency of the wireless power transmission at the first operating frequency, and if the efficiency falls below a threshold, then transmit wireless power transmission at a second operating frequency different to the first operating frequency, wherein the transmitter and receiver are configured to operate in any of regions; near-field, mid-field and far-field.

The transmitter 1200, 1400 can be the transmitter 200 or transmitter 400 described with reference to figures 2 and 4, and so transmitter 1200, 1400 may comprise any combination of the features of transmitter 200 or any combination of the features of transmitter 400. The receiver 1300 can be receiver 300 described with reference to figure 3, and so the receiver 1300 may comprise any combination of the features of the receiver 300. This will become apparent from the below description.

An indication of the wireless power transmission efficiency may comprise power information, or it may comprise an efficiency value, as described with reference to figures 2 to 9. Thus, in one example the receiver 1300 is configured to determine power information comprising a value of the power received from the wireless power transmission at the first operating frequency, and send the power information to the transmitter 1200, 1400, the transmitter 1200, 1400 being further configured to calculate the efficiency of the wireless power transfer based on the power information. In another example, the receiver 1300 is configured to determine power information comprising a value of the power received from the wireless power transmission at the first operating frequency, and also calculate the efficiency of the wireless power transfer based on the power information, and then send the efficiency to the transmitter 1200, 1400.

In another example the receiver 1300 comprises an electronic circuit 1325 having a processor 1330 and a memory 1335, which may correspond to the electronic circuit 325, processor 330 and memory 335 of receiver 300. The transmitter comprises an electronic circuit 1225, 1425, having a processor 1230, 1430 and a memory 1235, 1435 corresponding to the electronic circuit 225, 425, processor 230, 430 and memory 235, 435 of transmitter 200 or transmitter 400. In this example, the processor 1330 of the receiver 1300 is configured to determine power information comprising a value of the power received from the wireless power transmission at the first operating frequency, and send the power information to the transmitter 1200, 1400. The processor 1230, 1430 of the transmitter 1200, 1400 is further configured to calculate the efficiency of the wireless power transfer based on the power information. In another example, the processor 1330 of the receiver 1300 is configured to determine power information comprising a value of the power received from the wireless power transmission at the first operating frequency, and also calculate the efficiency of the wireless power transfer based on the power information, and then send the efficiency to the transmitter 1200, 1400.

In one example, the transmitter 1200, 1400 comprises a communication unit 1220, 1420. The communication units may correspond to either of the communication units 220, 420 described with reference to figures 2 and 4. The receiver 1300 may also comprise a communication unit 1320, which may correspond to the communication unit 320 of figure 3. The communication units of the transmitter 1200, 1400 and the receiver 1300 may be used for exchanging information such as power indication or efficiency as described in the preceding paragraph as well as above with reference to figures 2-4.

In one example, the receiver 1300 is configured to periodically send the power information, or the efficiency, to the transmitter over a wireless communication interface so that the operating frequency can be continuously adjusted. In one example, the processor 1330 of the receiver 1300 is configured to periodically send the power information, or the efficiency to the transmitter over a wireless communication interface so that the operating frequency can be continuously adjusted. The wireless communication interface may comprise the communication unit 1220, 1420 of the transmitter 1200, 1400 and the communication unit 1320 of the receiver 1300.

In one example, the transmitter 1200, 1400 comprises an adaptive gain control circuit 1210, 1410, for determining if the efficiency falls below the threshold. The adaptive gain control circuit 1210, 1410 may further be configured to determine whether to increase or decrease transmit power for transmitting the wireless power transmission at the first operating frequency based on the efficiency relative to the threshold. The adaptive gain control circuit 1210, 1410 may be configured to determine the second operating frequency based on the efficiency relative to the threshold. In one example, the adaptive gain control circuit 1210, 1410 correspond to the adaptive gain control circuits 210, 410 described with reference to figure 2 and 4.

Once the adaptive gain control circuit 1210, 1410 has determined to change transmit power and/or operating frequency, the adaptive gain control circuit 1210, 1410 is configured to send an instruction to the processor 1230, 1430 which in turn is configured to instruct a voltage-controlled oscillator to change operating frequency of a transmission antenna, as is explained below, and/or to increase the transmit power of the transmission antenna.

In one example, the transmitter 1200, 1400, or more specifically the adaptive gain control circuit 1210, may be configured such that prior to determining to transmit the wireless power transmission at a second operating frequency, increase the transmit power for transmitting the wireless power transmission at the first operating frequency.

In one example, the transmitter 1200, 1400 comprises a transmitter antenna 1205, 1405. The transmitter antenna 1205, 1405 is for transmitting radio waves of the frequency range of 30MHz to 12GHz. The transmitter antenna 1205, 1405 may be the transmitter antenna shown in figure 5 or figure 6. In another example, the receiver 1300 comprises a receiver antenna 1305. The receiver antenna 1305 is for receiving radio waves of the frequency range of 30MHz to 12GHz. The receiver antenna 1305 may be the receiver antenna shown in figures 7 to 9.

In one example, the transmitter 1400 further comprises a switching circuit 1415. The switching circuit 1415 is for switching resonance frequency of a transmitter antenna. More particularly, the switching circuit 1415 is used when the transmitter 1400 comprises a transmitter antenna having a modular configuration that can be activated or deactivated, for example the transmitter antenna shown in figure 6. The switching circuit is configured to enable and disable different parts of the transmitter antenna geometry by activating or deactivating pin diodes connecting a radiator patch with two ground patches of the transmitter antenna. This is to change the resonance frequency of the transmitter antenna for wireless power transfer as determined by the adaptive gain control circuit. In one example, the switching circuit 1415 correspond to the switching circuit 415 described with reference to figure 4.

In one example, the receiver 1300 may further comprise a converter 1310 for converting received radio frequency to direct current. This may also be referred to as a RF to DC circuit. As the receiver antenna 1305 receives the radio waves in the operating frequency of the transmitter 1200, 1400 alternating current is produced in the receiver antenna 1305. The converter 1310 is configured to convert the alternating current to direct current. The value or measurement of the direct current received is used as power information which may be sent to the transmitter 1200, 1400 to indicate the power received by the receiver 1300. The transmitter 1200 may use the power information to calculate or determine efficiency of the wireless power transfer as described above. In one example, the converter 1310 correspond to the converter 310 described with reference to figure 3.

In one example, the receiver 1300 further comprises a rechargeable battery 1315. The rechargeable battery is configured to store the energy received from the wireless power transfer. In one example, the battery 1315 correspond to the battery 315 described with reference to figure 3.

It should be understood that the receiver 1300 does not have to comprise a rechargeable battery, instead the receiver 1300 can be electrically connected to an implantable medical device comprising a rechargeable battery and the receiver 1300 being configured to send the energy to the rechargeable battery.

In one example, the transmitter 1200, 1400 and the receiver 1300 are configured to operate in the following frequency bands; the ultra-high frequency band (UHF), L-band, S-band, C-band and X-band.

In one example, the transmitter 1200, 1400 further comprises a voltage-controlled oscillator which upon instruction from the processor 1230, 1430 is configured to change the operating frequency of the transmitter antenna 1205, 1405.

In one example, the receiver 1300 is configured to receive transmitted wireless power transmission from the transmitter 1200, 1400 over a distance from 1cm to 100cm.

In one example, the receiver 1300 forms part of an implantable medical device. The implantable medical device may be a neurostimulation device.

It should be understood that the disclosure is not only directed to a system for wireless power transfer, but also to the receiver 300, 1300, and to the transmitter 200, 400, 1200, 1400, transmitter antenna 205, 405, 500, 600, 1205, 1405 and receiver antenna 300, 700, 1305. Additional configurations of the antennas are described below.

In one example, a transmitter antenna is provided. The transmitter antenna comprises a radiator patch and two ground patches located on a substrate such that the radiator patch and ground patches are coplanar, each ground patch being connected to the radiator patch via a pin-diode switch such that the radiator patch can be electrically connected to each ground patch independently. An implementation of the transmitter antenna may be the transmitter antenna 600 shown in figure 6.

In one example, the radiator patch and the ground patches are block shaped and the ground patches partially surround the radiator patch.

In one example, another transmitter antenna is provided. The transmitter antenna for transmitting wireless power transmission to a receiver in near-field, mid-field and far-field. The transmitter antenna comprises a radiator patch positioned on one side of a substrate, and a ground patch positioned on an opposing side of the substrate, and the radiator patch having a meandering end opposing a narrowing end. An implementation of the transmitter antenna may be the transmitter antenna 500 shown in figure 5.

Any of the transmitter antennas described herein may be configured to transmit wireless power transmission in the following frequency bands; the ultra-high frequency band (UHF), L-band, S-band, C-band and X-band.

In one example, a transmitter is provided comprising any of the transmitter antennas described herein. Examples of the transmitter may be transmitter 200, 400, 1200 or 1400.

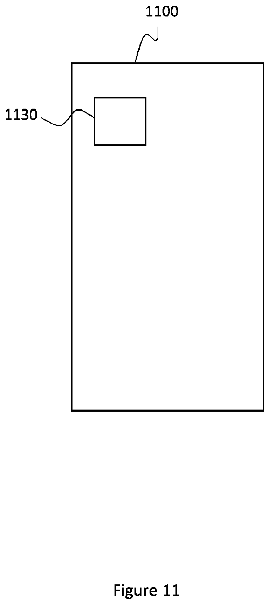

Figure 11 shows a schematic illustration of a charging unit 110 comprising a transmitter 1130. The transmitter 1130 may be any of the transmitters described herein. The charging unit can be a wearable device, for example, it can be a wrist watch or it can be configured to be carried in a pocket of clothing. The charging unit may comprise a rechargeable battery (not shown). In another example, the charging unit is a stationary unit. The charging unit may also be referred to as an External Power Unit (EPU).

In one example, a receiver antenna for receiving wireless power transmission from a transmitter in near-field, mid-field and far-field is provided. The receiver antenna comprises an inner helix located on a disc, and an outer helix surrounding the inner helix, wherein the outer helix comprises electrical terminals. An implementation of the receiver antenna may be the receiver antenna 700 shown in figures 7 to 9.

In one example, a receiver is provided comprising the receiver antennas described herein. Examples of the receiver may be receiver 300 and receiver 1300.

In one example, an implantable medical device is provided comprising a receiver as described herein.

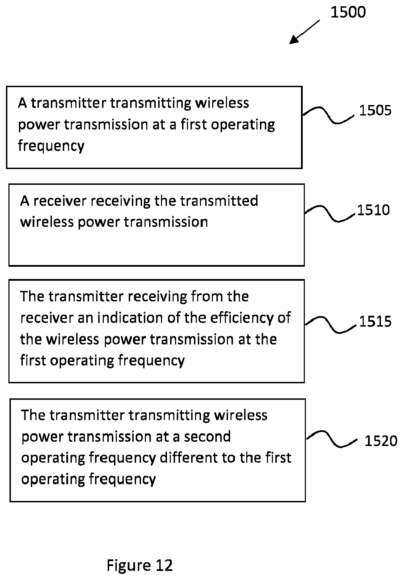

An example of a method 1500 performed by a power transfer system will now be described with reference to figure 12. The method may be performed by any of the power transfer systems described or any of the transmitters and receivers described herein.

The method comprises a transmitter transmitting wireless power transmission at a first operating frequency, operation 1505. A receiver receiving the transmitted wireless power transmission, operation 1510. The transmitter receiving from the receiver an indication of the efficiency of the wireless power transmission at the first operating frequency, operation 1520. If the efficiency falls below a threshold, then the method further comprises the transmitter transmitting wireless power transmission at a second operating frequency different to the first operating frequency, operation 1525. The transmitter and the receiver are configured to operate in any of regions; near-field, mid-field and far-field.

In one example, the method 1500 further comprises the receiver measuring a value of direct current received through the wireless power transmission at the first operating frequency, and the receiver transmitting the value as an indication of the efficiency of the wireless power transmission to the transmitter. The value may also be referred to as power information.

In one example, the transmitter determines the efficiency based on the power information.

In one example, operations 1505 to 1520 are repeated periodically, for example every 0.1, 1 or 60 seconds.

In one example, the transmitter compares the efficiency with a threshold value, and if the efficiency falls below the threshold value, the transmitter changes the transmit power and/or operating frequency.

In one example, the transmitter first attempts to increase the transmit power of a transmitter antenna of the transmitter and if based on an updated power information received from the receiver after the transmit power has been increased the efficiency value still falls below a threshold, the transmitter may proceed with changing the operating frequency of the transmitter antenna 205 to the second operating frequency. As mentioned above, this process may be continuously repeated in order to maintain the efficiency of the wireless power transfer or to find an optimal transmit power and/or frequency, which may be needed if the distance and/or orientation between the transmitter and receiver changes due to movement of the user relative to the transmitter.

In an alternative example on how the transmitter handles the efficiency, the transmitter compares the efficiency with a plurality of predetermined ranges of efficiency, each range relating to a transmit power and operating frequency. Thus, when the efficiency is determined to fall within a predetermined range associated with a transmit power and operating frequency, the transmitter changes the transmit power and operating frequency of the transmitter antenna accordingly. This process may be continuously repeated to adapt to the changing distance between the transmitter and receiver due to movement of the user relative to the transmitter.

In yet another example, it may be that the transmitter proceeds to change the operating frequency when the efficiency falls above a threshold value. For example, the operating frequency can be changed to lower the energy used in order to save battery or to limit the transmitter output power to remain below SAR limit of 1.6W/Kg.

In one example, when the transmitter determines that the first operating frequency needs to change to the second operating frequency, an adaptive gain control circuit of the transmitter signals to a processor of the transmitter which in turn instructs a voltage-controlled oscillator of the transmitter connected to a transmitter antenna of the transmitter to increase or decrease the oscillation of the current in order to achieve the desired second frequency to be transmitted by the transmitter antenna.

In another example, where the transmitter and the receiver are used for wireless power transfer, the transmitter changes the resonance frequency of its antenna by employing its switching circuit 415 to activate and/or deactivate relevant pin-diodes of the transmitter antenna. Thereafter, the transmitter changes the operating frequency to the second operating frequency using the voltage-controlled oscillator as described above.

In the above described examples, the receiver is configured to determine power information which indicates a value of the power received from the transmission of radio waves from the transmitter, and the transmitter is configured to determine an efficiency (or efficiency value) based on the power information. However, it should be understood that the receiver can instead be configured to also determine the efficiency based on the power information and then send the efficiency over to the transmitter such that the transmitter then compares the received efficiency with a threshold and/or predetermined ranges.

The above described examples enable wireless power transfer to be continuously above a certain efficiency which allows for the distance and/or orientation of the receiver and transmitter to be continuously changed. This means that users of implanted medical devices comprises one of the example receivers described herein has greater flexibility of movement without the loss of power transfer from the transmitter.

The above described examples are suitable for implantable medical devices such as neurostimulation devices.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification, and the contents of all such papers and documents are incorporated herein by reference.

## Claims

1. A wireless power transfer system for an implantable medical device, the system comprising;
a transmitter (200, 400, 1200, 1400) configured to transmit wireless power transmission at a first operating frequency,
a receiver (300, 1300) configured to receive the transmitted wireless power transmission, and to be electrically connectable to an implantable medical device,
the transmitter being further configured to;
receive from the receiver an indication of an efficiency of the wireless power transmission at the first operating frequency, and if the efficiency falls below a threshold, then
transmit wireless power transmission at a second operating frequency different to the first operating frequency, wherein
the transmitter and receiver are configured to operate in any of regions; near-field, mid-field and far-field.

2. The wireless power transfer system according to claim 1, wherein the transmitter (200, 400, 1200, 1400) and the receiver (300, 1300) are configured to operate in the following frequency bands; the ultra-high frequency band (UHF), L-band, S-band, C-band and X-band.

3. The wireless power transfer system according to any preceding claim, wherein the transmitter (200, 400, 1200, 1400) comprises a transmitter antenna as claimed in any of claims 12 to 17, wherein the transmitter is configured to transmit the wireless power transmission.

4. The wireless power transfer system according to any preceding claim, wherein the receiver (300, 1300) comprises a receiver antenna as claimed in any of claims 19 to 22, wherein the receiver is configured to receive the wireless power transmission.

5. The wireless power transfer system according to any preceding claim, wherein the transmitter (200, 400, 1200, 1400) is configured to, prior to transmitting wireless power transmission at a second operating frequency, increase the transmit power for transmitting the wireless power transmission at the first operating frequency.

6. The wireless power transfer system according to any preceding claim, wherein the transmitter (200, 400, 1200, 1400) is configured to determine the second operating frequency based on the efficiency.

7. The wireless power transfer system according to any preceding claim, wherein the receiver (300, 1300) is configured to determine power information comprising a value of the power received from the wireless power transmission, and send the power information to the transmitter, the transmitter (200, 400, 1200, 1400) being further configured to determine the efficiency of the wireless power transfer based on the power information.

8. The wireless power transfer system according to claim 7, wherein the receiver (300, 1300) is configured to periodically send the power information to the transmitter (200, 400, 1200, 1400) over a wireless communication interface so that the operating frequency can be continuously adjusted.

9. The wireless power transfer system according to any preceding claim, wherein the receiver (300, 1300) is configured to receive transmitted wireless power transmission from the transmitter (200, 400, 1200, 1400) over a distance from 1 cm to 100cm.

10. The wireless power transfer system according to any preceding claim, wherein the receiver (300, 1300) forms part of an implantable medical device.

11. The wireless power transfer system according to claim 10, wherein the implantable medical device is a neurostimulation device.

12. A transmitter antenna (600) for transmitting wireless power transmission to a receiver in near-field, mid-field and far-field, the transmitter antenna comprises a radiator patch (605) and two ground patches (610, 615) located on a substrate such that the radiator patch and ground patches are coplanar, each ground patch being connected to the radiator patch via a pin-diode (620, 625) switch such that the radiator patch can be electrically connected to each ground patch independently.

13. The transmitter antenna as claimed in claim 12, wherein the substrate comprises a glass-reinforced epoxy resin laminate, and the radiator patch (605) and ground patches (610, 615) comprise copper.

14. The transmitter antenna according to any of claims 12 or 13, wherein the radiator patch (605) and the ground patches (610, 615) are block-shaped and the ground patches partially surround the radiator patch.

15. The transmitter according to any of claims 12 to 14, wherein the substrate is 35mm wide, 35mm long, and 1mm thick.

16. A transmitter antenna (500) for transmitting wireless power transmission to a receiver in near-field, mid-field and far-field, the transmitter antenna comprises a radiator patch (505) located on one side of a substrate, and a ground patch located on an opposing side of the substrate, and the radiator patch having a meandering end (520) opposing a narrowing end (525).

17. The transmitter antenna (500, 600) according to any of claims 12 to 16, wherein the transmitter antenna being configured to transmit wireless power transmission in the following frequency bands; the ultra-high frequency band (UHF), L-band, S-band, C-band and X-band.

18. A charging unit (1100) comprising the transmitter antenna (500, 600) as claimed in any of claims 12 to 17.

19. A receiver antenna (700) for receiving wireless power transmission from a transmitter in near-field, mid-field and far-field, the receiver antenna comprising an inner helix (705) located on a disc (710), and an outer helix (715) surrounding the inner helix, wherein the outer helix comprises electrical terminals.

20. The receiver antenna according to claim 19, wherein the outer helix (715) extends in an axial direction beyond that of the inner helix (705).

21. The receiver antenna according to claims 19 or 20, wherein the outer helix (715) is positioned on or around the disc (710).

22. The receiver antenna according to any of claims 19 to 21, wherein the outer helix (715) is 5.5mm in height and has a diameter of 3mm, and the inner helix (705) is 3mm in height and has a diameter of 1.5mm.

23. An implantable medical device comprising a receiver antenna as claimed in any of claims 19 to 22.
